# EUROPEAN PATENT APPLICATION

(11) **EP 1 452 589 A1**
(43) Date of publication of application: **01.09.2004**
(21) Application number: 03004346.7
(22) Date of filing: 28.02.2003
(51) Int. Cl.: C12N 9/10, C12N 15/62, C12N 15/54, C12N 5/10, A61K 38/45, C07K 14/21

(54) **ADP-ribosyltransferase c3cer**

(71) Applicant: Migragen AG, 72076 Tübingen (DE)
(72) Inventor: Aktories, Klaus, Prof. Dr. Dr., 79189 Bad Krozingen (DE); Wilde, Christian, Dr., 79106 Freiburg (DE)
(74) Representative: Bösl, Raphael, Dr.rer.nat., Dipl.-Chem.

(57) **Abstract**

The present invention relates to the ADP-Ribosyltransferase C3cer and its use for the preparation of a pharmaceutical composition for the regeneration of neurons and/or the stimulation of the growth of neurons.

## Description

The present invention relates to the ADP-Ribosyltransferase C3cer and its use for the preparation of a pharmaceutical composition for the regeneration of neurons and/or the stimulation of the growth of neurons.

The nervous system of vertebrates is divided into the central nervous system (CNS) and the peripheral nervous system (PNS). Brain and Spinal cord form the CNS, while the remaining neuronal pathways form the PNS.

Generally, the nervous system is composed of neurons and glia cells. In the CNS, the glia cells are oligodendrocytes and astrocytes. The oligodendrocytes form the myelin sheet around the neuronal extensions, mainly the axons. In the PNS, the Schwann cells surround the axons of the neurons and contribute e.g. to the velocity of the signal transmission along the axon.

While the central nervous system exhibits high plasticity, i.e. a high ability to change and make new connections, it has very little regenerative capacity. In contrast, the peripheral nervous system, especially the motor neurons, has regenerative powers, even in adult mammals. The regeneration of motor neurons involves regrowing a severed axon, not replacing a missing or diseased cell body. If the cell body of a motor neuron is destroyed, it cannot be replaced.

Since the neurons of the central nervous system cannot regenerate their axons under normal conditions, spinal cord injuries often have serious consequences and often cause permanent paralysis. Furthermore, the destruction or neurons in neurological and neurodegenerative diseases of the central nervous system is often irreversible.

In the past years, many efforts have been taken in order to elucidate the molecular reasons for the inability of the CNS neurons to regenerate.

One strategy is to find ways of enlarging the population of neural stem cells and to direct their development in ways that circumvent the lesions caused by disease or trauma. The neural stem cells found in adult mammals may be very similar to embryonic neural stem cells and can respond to the same growth factors (Johe KK et al., 1996, Genes Dev. 10:917-927; Johansson FB et al., 1999, Cell 96:291-302).

Another strategy for CNS neural regeneration is to create environments that encourage axonal growth. Unlike the Schwann cell of the peripheral nervous system, the myelinating cell of the central nervous system, the oligodendrocyte, produces substances that inhibit axon regeneration (Schwab ME, Caroni P, 1988, J. Neurosci. 8:2381-2393).

A further strategy is to influence the signal transduction pathways of neurons. It is known that the Ras-related Rho-family p21 GTPases Cdc42, Rac and Rho participate in signalling pathways influencing growth cone (the growing end of the axon) and morphology in neuroblastoma cells (Kozma R et al., 1997, Mol. Cell Biol. 17(3):1201-1211). For example, Rac activates the neutrophil oxidative burst (Abo A et al., 1991, Nature (London) 353:668-670). In particular, these GTPases have been shown to induce morphological changes associated with actin polymerization.

It is known that the activation of several Rho family GTPases, namely RhoA, RhoB and RhoC, leads to a strong inhibition of the growth of nerve fibres under cell culture conditions (Mueller BK, 1999, Annu. Rev. Neurosci. 22: 351-388). Experimental data indicate that specific activation of RhoA, B, C inside of the nerve fibre represents an essential mechanism of inhibition of the growth of nerve fibres (Jin Z and Strittmatter SM, 1997, J. Neurosci. 17: 6256-6263; Lehmann M, et al., 1999, J. Neurosci. 19: 7537-7547; Wahl S et al., 2000, J. Cell. Biol. 149: 263-270). Activation of RhoA, B and C leads to a collapse of the growth cones in new developing nerve fibres, which results in the inhibition of the formation of new nerve pathways.

In a mouse animal model of spinal cord injury a Rho antagonist induces functional recovery (Dergham P et al., 2002 J. Neurosci. 22:6570-6577).

Furthermore, Rho is an important target for the treatment of cancer and metastasis (Clark et al., 2000, Nature 406:532-535) and hypertension (Uehata et al., 1997, Nature 389: 990) and Rho is reported to have a cardioprotective role (Lee et al., 2001, FASEB J. 15:1886-1884).

It is known in the art that RhoA, B, C can be inhibited by ADP-ribosyltransferases. These enzymes transfer a ADP-ribose to RhoA, B, C, which results in an inhibition of the function of the protein (Aktories, K et al., 1992, Curr. Top. Microbiol. Immunol. 175:115-131; Sekine, A et al., 1989, J. Biol. Chem. 264, 8602-8605; Braun, U et al., 1989, FEBS Lett. 243, 70-76.

A typical example for this type of enzymes is the family of C3-like ADP-ribosyltransferases (Wilde, C and Aktories, K, 2001, Toxicon 39:1647-1660; Aktories, K et al., 2000, in Handbook of Experimental Pharmacology pp 207-233, Springer Verlag, Berlin). It comprises, e.g., the prototype C3bot (Aktories, K et al., 1987, FEBS Lett. 212, 109-113; Rubin, E J et al., 1988, Mol. Cell. Biol. 8, 418-426; Rösener, S et al., 1987, FEBS Lett. 224, 38-42), including the isoforms C3bot1 and C3bot2 from *Clostridium botulinum,* the C3lim from *Clostridium limosum* and various transferases from *Staphylococcus aureus* including C3stau1, formerly known as EDIN A (Inoue, S et al., 1991, Biochem. Biophys. Res. Commun. 174, 459-464), C3stau2 (EDIN B) (Wilde, C et al., 2001, J. Biol. Chem. 276, 9537-9542) and C3stau3 (EDIN C) (Yamaguchi, T et al., 2001, Infect. Immun. 69, 7760-7771). The members of this family share only limited sequence similarity, but key amino acid residues and short peptide stretches are conserved in all of them.

The C3-like ADP-ribosyltransferases known in the art are able to modify and thus, to change the activity of small GTP-binding proteins, however, they are not able to selectively change the activity of one or more selected GTP-binding protein(s).

Thus, for example, C3bot and C3lim apart from RhoA, RhoB and RhoC, also ADP-ribosylate Rac1 and, in the case of C3lim, also Cdc42.

However, because of the known interactions between Rho-, Rac- and Cdc42-signal transduction pathways (Mueller BK, 1999, Annu. Rev. Neuroscience, 22:351-388), the ADP-ribosylation of diverse small GTP-binding proteins leads to contraries effects. For example Rac1 and Cdc42Hs stimulate formation of axons, while RhoA induces growth cone collapse. It is suggested that a switching between activation of Cdc42Hs and Rac1 or RhoA is a mechanism to regulate either extension or retraction of the growth cone (Kozma R et al., 1997, Mol. Cell Biol., 17(3):1201-1211). Consequently, the ADP-ribosylation of various small GTPases leads to contrary effects.

A further example is that C3stau1 and C3 stau2 ADP-ribosylate RhoA, RhoB and RhoC, but also RhoE and Rnd3. RhoE belongs to a Rho subfamily, so called the Rnd proteins. RhoE exhibit notable functional differences from RhoA, which are opposed to that of RhoA. For example, RhoE stimulates the formation of filopodia-like cellular structures and induces dissolution of stress fibres and loss of cell adhesion. RhoE can be described as a functional RhoA antagonist (Guasch et al., 1998, Mol. Cell. Biol., 18:4761-71; Nobes et al., 1998, J. Cell Biol., 141:187-197).

Thus, the ADP-ribosylation of RhoA, B, C is constricted by the simultaneous APD-ribosylation of other Rho family GTPases.

Further modulators of the activity of small GTPases are known. (Ozdinler PH, and Erzurumlu RS, 2001, Comp. Neurol., 438:377-387; Arthur WT, et al., 2002, Biol. Res. 200, 35:239-246). However, none of these selectively inhibit RhoA, B or C activity.

Consequently, in the art it is not possible to selectively inhibit Rho A, B, C. The known C3 ADP ribosyltransferases modify apart from RhoA, B, C also other small GTPases, which impairs neuronal growth. However, such means are highly needed to overcome the strong constricted effects of the ADP-ribosylation of RhoA, B, C caused by the simultaneous APD-ribosylation of other Rho family GTPases.

The problem of the present invention is therefore to provide a mean which can selectively inhibit RhoA, B, C, thereby permitting an effective regeneration of neurons and stimulation of neuron growth.

This problem is solved by the ADP-ribosyltransferase C3cer.

Surprisingly a new ADP-ribosyltransferase, so called ADP-ribosyltransferase C3cer, could be identified, which selectively ADP-ribolysates RhoA, RhoB and RhoC.

In the context of the present invention the term "C3cer" relates to an ADP-ribosyltransferase which selectively ADP-ribosylates and therefore inhibits RhoA, B, C. C3cer relates to a distant subfamily of C3-like ADP-ribosyltransferases.

According to the present invention the term "ADP-ribosylates" or "ADP-ribosylation" means transferring the ADP-ribose moiety of NAD onto an acceptor amino acid of a protein.

According to the present invention the term "selective ADP-ribosylation" means that RhoA, B or C, preferably all three of them, but not RhoE, Rnd3 and Cdc42 are ADP-ribosylated.

According to the present invention the term "inhibiting" or "inhibit" means the modification of the biological activity of one or more molecule(s), in particular of small GTP-binding proteins, in such a way that their activity is essentially suppressed.

According to the present invention C3cer can be obtained from *Bacillus cereus,* but the invention is not limited to a C3cer of this organism. ADP-ribosyltransferase C3cer which shows the same biological activities and features are herewith included. Methods for isolating proteins from bacteria are well known in the art (Just I et al., 1995, Biochem., 34(1):334-340; Aktories K et al., 1988, Biochem., 172(2):445-450).

A method for identifying an ADP-ribosyltransferase C3cer is to incubate a substrate, usually proteins in cell lysates, e.g., brain tissue homogenate, fibroblast culture or platelet lysate, with a potential ADP-ribosyltransferase C3cer in the presence of radioactively labeled [³²P]NAD. Radioactively labeled proteins, which are modified by ADP-ribosylation of the ADP-ribosyltransferase C3cer can be identified by SDS polyacrylamid electrophoresis and subsequent autoradiography or phosphor-imaging. Thus, the radiolabeled proteins can be analyzed and the protein to be analyzed is characterized as an ADP-ribosyltransferase.

A method for testing the selective ADP-ribosylation of the potential ADP-ribosyltransferase C3cer - and therefore identifying it as a C3cer - is to use the Rho GTPases RhoA, RhoB, RhoC, Cdc42, RhoE, and Rnd3 as a protein substrate for an incubation with the potential ADP-ribosyltransferase C3cer in the presence of radioactively labelled [adenylate-³²P]NAD. After the incubation, the amount of incorporated [³²P]ADP-ribose can be calculated from phosphor-imager data for the single Rho GTPases. Such an *in vitro* ADP-ribosylation assay can be carried out as described in Example 5 below. If the ADP-ribosyltransferase selectively ADP-ribosylates RhoA, RhoB and RhoC, then the ADP-ribosyltransferase tested is a C3cer.

In a preferred embodiment the ADP-ribosyltransferase C3cer contains or consists of the amino acid sequence of SEQ ID NO: 1. The ADP-ribosyltransferase C3cer of SEQ ID NO: 1 has 219 amino acids, a mass of 25.242 kDa and a theoretical isoelectric point of 9.30.

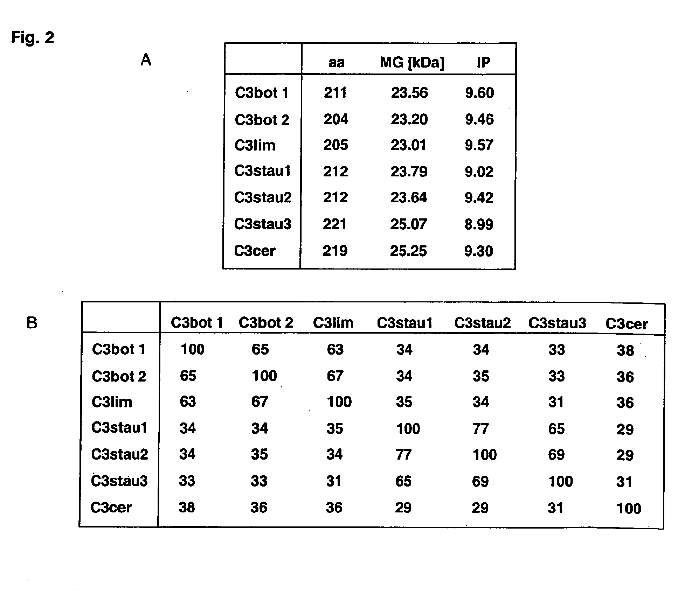

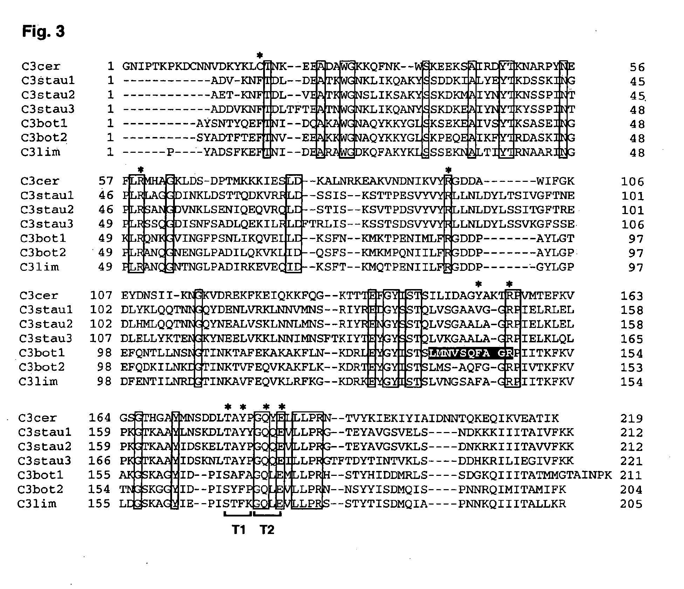

In comparison with other C3-like ADP-ribosyltransferases as depicted in SEQ ID:NO: 1 the C3cer exhibits the lowest sequence amino acid identity to all other C3-like ADP-ribosyltransferases (see Fig. 2B, 3).

Further, the term "ADP-ribosyltransferase C3cer" also relates to active variants of an ADP-ribosyltransferase C3cer with the amino acid sequence of SEQ ID NO: 1.

The term "active variant of an ADP-ribosyltransferase C3cer" according to the invention means an ADP-ribosyltransferase C3cer which selectively ADP-ribosylates RhoA, B, C but which does not have the sequence of SEQ ID NO: 1. Preferably, such an active variant contains or consists of an amino acid sequence exhibiting a sequence homology of at least about 70%, preferred of at least about 80%, more preferred of at least about 90% and most preferred of at least about 95% to the amino acid sequence shown in SEQ ID NO: 1.

The active variant can be identified and tested as described above.

In a preferred embodiment C3cer is incorporated in a target cell.

The term "incorporation into a cell", or "inserting into a cell" relates also to the import of a protein, preferably the ADP-ribosyltransferase C3cer, a nucleic acid or parts thereof. For this, several methods are known, e.g., permeabilization of cells, microinjection of proteins, transfection of suitable vectors or toxins, osmotic shock, lipid carriers.

According to the present invention the term "target cell" means a cell in which the ADP-ribosyltransferase C3cer should be incorporated. Preferably, the target cell is a peripheral or central neuron.

In a further subject matter the present invention relates to a fusion protein which contains the ADP-ribosyltransferase C3cer of the invention and at least a second protein.

According to the present invention the term "fusion protein" relates to a polypeptide which comprises at least two components descending from different proteins, whereby the single polypeptide chains are bound covalently to each other. Preferably, such a polypeptide comprises at least about 50 amino acids, preferred at least about 60 amino acids, more preferred at least about 70 amino acids.

The term "second protein" according to the present invention relates to a protein or a peptide which can bind to a structure of the surface of a cell or to another molecule, preferred a protein or a peptide, more preferred a transporter molecule.

Preferably, the second protein contains or consists of the enzyme component of a binary bacterial protein toxin or peptides thereof. Examples for such binary bacterial protein toxins are C2 toxin from *Clostridium botulinum* or anthrax toxin from *Bacillus anthracis* (Frankel AE et al., 2002, Curr. Protein Pept. Sci, 4:399-407), iota toxin from *Clostridium perfringens* (Marvaud J-C et al., 2002, J. Biol. Chem., 277(46):43659-43666) or the transferases Toxin A and B from *Clostridium difficilis.*

In a particularly preferred embodiment, the second protein mediates the incorporation of the fusion protein into a target cell.

According to the present invention the term "incorporation into a cell", or "inserting into a cell" means also to import a fusion protein inside a cell. Preferably, the second protein mediates the incorporation into the target cell by binding to a structure on the cell surface.

Preferably, the incorporation into a cell occurs by endocytose, more preferably by an endocytose which is mediated by a binary bacterial toxin.

In a preferred embodiment the second protein binds directly to the target cell.

In a preferred embodiment the second protein binds via a transporter to the target cell.

A "transporter" according to the present invention relates to a molecule which binds first to the second protein and then to a structure on the surface of a cell. A preferred transporter contains a binding component which binds to the second protein.

In a preferred embodiment, the second protein is part of a binary molecule comprising at least two subunits. The other subunit is used as a transporter in order to bring the fusion protein to the cell surface.

For example, such a binding via the transporter can be mediated by a binary bacterial protein toxin. In a preferred embodiment the binary bacterial protein toxin contains an enzyme component and a binding component. A preferred binary bacterial protein toxin is the binary actin-ADP-ribosylating C. *botulinum* C2 toxin which consists of the enzyme (active) component C2I and the binding (translocation) component C2II which are separate protein subunits. The active component C2I enters cells through C2II by receptor-mediated endocytosis and membran translocation. For this purpose C2II binds to the surface of the target cell, thereby inducing a binding site for C2I and subsequently mediating the internalization and membrane translocation of the enzyme component C2I. C2I possess ADP-ribosyltransferase activity and modifies selectively G-actin. The N-terminal part of C2I (C2IN) consists of 225 amino acid residues. It lacks ADP-ribosyltransferase activity and acts as the C2II contact site.

Therefore, in a particularly preferred embodiment the second protein of the invention contains or consists of an enzyme component of a binary bacterial protein toxin, preferred the C2I component, more preferred the C2IN component of the C2 toxin, preferred the C2 toxin from *C. botulinum.* Said C2IN component may bind via a transporter, e.g. the C2II component of the C2 toxin, to the cell surface. However, the preferred fusion protein comprising the C3cer of the invention and a C2I component may also bind directly to the cell and may then be incorporated.

Therefore, in a preferred embodiment the transporter of the invention contains or consists of the binding component of a binary bacterial toxin, preferred the C2II component of the C2 toxin, preferred of the C2 toxin from *C. botulinum.*

In a further preferred embodiment the transporter binds to a structure on the surface of the target cell, particularly to a receptor or a surface protein. According to the invention such a structure of the target cell can be, e.g. a carbohydrate, preferably a asparagine-linked carbohydrate (Eckhardt M et al., 2000, J Biol Chem, 275(4):2328-34).

In preferred embodiments the second protein is diphtheria toxin (DT) from *Corynebacterium diphteriae;* pseudomonas exotoxin A (PEA) from *Pseudomonas aeruginosum* (Lemichez E et al., 1997, Mol Microbiol., 23(3):445-457); Tat or antennapedia or Bac7 (Sadler et al., 2002, Biochemistry 41:14150-14157); or Poly-Arginine, ASPT (Wintson et al., 2002, J Biol. Cem. Sep 6, 277(36):32820-32829).

Since the fusion protein contains C3cer, it selectively ADP-ribosylates RhoA, RhoB and/or RhoC and therefore inhibits RhoA, RhoB and/or RhoC.

Another preferred embodiment relates to the fusion protein containing or consisting of the amino acid sequence of SEQ ID NO: 2.

This amino acid sequence consists of the ADP-ribosyltransferase C3cer according to the invention and the N-terminal part (C2IN) of the enzyme component C2I of the C2 toxin from *C. botulinum.*

Furthermore, the invention comprises active variants of the fusion protein showing the amino acid sequence of SEQ ID NO: 2.

The term "active variant of the fusion protein" according to the invention means a fusion protein which also ADP-ribosylates selectively RhoA, B, C, and which second protein mediates the incorporation into a cell essentially equally effective, but which sequence is not identical to the sequence given in SEQ ID NO: 2. Such an active variant contains or consists of an amino acid sequence showing an sequence homology of at least about 70%, preferred of at least about 80%, more preferred of at least about 90% and most preferred of at least about 95%.

A method for testing the effectiveness of an active variant compared to the fusion protein showing the amino acid sequence of SEQ ID NO: 2 can be carried out as follows: by addition antagonists that inactivate RhoA, B, C by ADP-ribosylation of the effector domain to living cells, said antagonists can be identified by detecting a molecular weight shift in Rho A, B, C. The molecular weight shift can be detected after the treatment of the cells with said Rho antagonists by homogenizing the cells, separating the proteins in the cellular homogenate by SDS PAGE. The proteins are transferred to nitrocellulose paper, Rho A, B, C are detected with Rho-specific antibodies by a Western blotting technique.

In another subject matter the invention relates to a nucleic acid which encodes the APD-ribosyltransferase C3cer or the fusion protein of the invention.

In preferred embodiments the nucleic acid contains or consists of the nucleic acid sequence SEQ ID NO:3 or SEQ ID NO:4 or SEQ ID NO:5.

In a further subject matter the present invention relates to a target cell which contains the ADP-ribosyltransferase C3cer, the fusion protein or the nucleic acid of the invention.

In a further subject matter the present invention relates to the use of the ADP-Ribosyltransferase C3cer or the fusion protein of the invention or the nucleic acid of the invention for the preparation of a pharmaceutical composition for the regeneration of neurons and/or for the stimulation of the growth of neurons, preferably in humans.

Preferably, the regeneration of neurons and/or the stimulation of growth of neurons is in the context of neurological and/or neurodegenerative diseases of the peripheral and/or central nervous system.

In a particularly preferred embodiment the disease is selected of the group comprising: stroke, multiple sclerosis, HIV dementia, Parkinson's disease, Alzheimer's disease, ischemia, epilepsy, Morbus Huntington ALS, neuronal regeneration after tumor extirpation, prion disease or other diseases of the CNS where axons are damaged in the CNS environment, diabetic neuropathy, cancer, metastasis, hypertension, cardiac diseases, hyperalgesia, neuropathy, eye diseases such as glaucoma, penile erectile dysfunction. Furhtermore the treatment with Rho antagonists would be used to enhance the rate of axon growth of peripheral nerves and thereby be effective for repair of peripheral nerves after surgery.

In another preferred embodiment, the regeneration of neurons and/or the stimulation of growth of neurons is in the context of lesions of the cerebrum and/or spinal cord.

In a further subject matter the present invention relates to a method for the production of a production cell comprising the insertion of at least one nucleic acid of the invention into a cell.

The term "production cell" according to the invention relates to any cell which is suitable to express the nucleic acid of the invention, e.g. a bacterial cell.

The insertion of a nucleic acid into a cell is well known in the art (see e.g. Sambrook, Fritsch, Maniatis, 2^{nd} edition 1989, Chapter 1, 4, 16.).

For example, a production cell containing the ADP-ribosyltransferase C3cer can be produced by the transformation of *E. coli* with a plasmid encoding C3cer-glutathione S-transferase fusion protein.

The invention also relates to a production cell obtainable by the above method.

In a further subject matter the present invention relates to a method for the production of the ADP-ribosyltransferase C3cer or the fusion protein of the invention, comprising the following steps:
a. cultivating a production cell of the invention and expressing the nucleic acid under suitable conditions, and
b. isolating the ADP-ribosyltransferase C3cer or the fusion protein with suitable means.

"Suitable conditions to express the nucleic acid" according to the invention are, for example, the induction of the expression by IPTG treatment, in case that the gene is under the control of the lac Z promoter.

The "isolation" of the ADP-ribosyltransferase C3cer or the fusion protein according to the invention can be carried out by the lyse (e.g., by French press) of the production cell, subsequent centrifugation and isolation of the protein from the supernatant by standard methods. For example, if C3cer-glutathione-S-transferase fusion proteins are to be expressed, these proteins can be purified using glutathione-Sepharose beads. After subsequent washing the ADP-ribosyltransferase C3cer or the fusion protein according to the invention will be released from the beads by thrombin treatment and, finally, thrombin can be removed by benzamidine beads. The procedure can be performed as described in Example 2 below.

In a further subject matter the present invention relates to a pharmaceutical composition containing the ADP-ribosyltransferase C3cer and/or the fusion protein of the invention and suitable adjuvants and/or pharmaceutically acceptable carriers.

The pharmaceutical composition of the invention may further comprise a transporter molecule. In this case, the transporter molecule and the ADP-ribosyltransferase C3cer and/or the fusion protein of the invention may be provided in one pharmaceutical composition or in separated pharmaceutical compositions.

Furthermore, the invention also relates to a pharmaceutical composition comprising the nucleic acid of the invention.

Suitable adjuvants and/or additives according to the invention are known in the art and include, but are not limited to 0,01-0,1 M, preferably 0,05 M phosphate buffer or 0,8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also present such as, for example, antimicrobials, antioxidants, collating agents, inert gases and the like.

Preferably, when administering the proteins of the invention, between 100 µg and 100 mg C3cer or fusion protein are administered.

A pharmaceutical composition according to the invention can be administered by routes known in the art, e.g., parental, paracanceral, transmuscosal, transdermal, intramuscular, intravenous, intradermal, subcutaneous, intraperitoneal, intraventricular, intrathecal, intracranial, intratumural, and preferred directly at a central nervous system lesion site or peripheral nervous system site.

In a further subject matter the present invention relates to a method for the production of a pharmaceutical composition according to the invention comprising the following steps:
a. performing the method for the production of the ADP-ribosyltransferase C3cer or the fusion protein as described above, and
b. combining the ADP-ribosyltransferase C3cer and/or the fusion protein with suitable adjuvants and/or additives.

The term "combining" according to the invention means to mix the ADP-ribosyltransferase C3cer or the fusion protein with suitable adjuvants and/or additives as described above.

The invention further relates to a method for the treatment of a patient, wherein a protein or nucleic acid of the invention are administered in a sufficient amount. Preferably, the patient suffers from a disease mentioned above. The proteins may be administered as mentioned above.

### Figures

Fig. 1 shows the DNA sequence of the ADP-ribosyltransferase C3cer from *Bacillus cereus* strain 2239. The deduced amino acid sequence is given in the upper lane. Peptides from tryptic digest of the purified native transferase are boxed.

Fig. 2 A) shows the properties of C3-like ADP-ribosyltransferases. aa: amino acids; IP: theoretical isoelectric point. Accession-numbers are: P15879 [C3bot1], Q00901 [C3bot2], X87215 [C3lim], P24121 [C3stau1], AJ277173 [C3stau2], AP003088 [C3stau3], AJ429241 [C3cer].
Fig. 2 B) shows the amino acid identity between C3-like ADP-ribosyltransferases. Sequences (without signal sequence). The different amino acids were aligned using the program "pair wise blast" at NCBI and the identity is given in %.

Fig. 3 shows the alignment of C3cer amino acid sequence with other C3-like ADP-ribosyltransferases. The numbers indicate the position of the residues in the whole protein. The conserved residues are boxed and an asterisk marks mutated residues in the C3cer sequence. The ADP-ribosylating toxin turn-turn ("ARTT")-motif is indicated under the C3lim sequence. **T1:** Turn1, **T2:** Turn2. The amino acid residues covering the phosphate-nicotinamide-loop (PN-Loop) are boxed in C3bot.

Fig. 4 shows the immunological relationship of C3cer to other C3-like ADP-ribosyltransferases. 200 ng of recombinant C3bot, C3lim, C3stau2 and C3cer were subjected to SDS-PAGE. Western-Blot analysis was performed with specific polyclonal antibodies as described in Example 8.

Fig. 5 A) shows the substrate specificity of several recombinant C3-like ADP-ribosyltransferases. 1 µg of the indicated GTPases (2 µM) were ADP-ribosylated by the indicated C3-like ADP-ribosyl-transferases (10 nM final, 5 min at 37°C for GST-RhoA or 200 nM final, 60 min at 37°C for other GST-GTPases) in the presence of [³²P] NAD. Radiolabeled probes were subjected to SDS-PAGE and the autoradiography is shown.
Fig. 5 B) shows the substrate specificity of recombinant ADP-ribosyltransferase C3cer. 1 µg of the indicated GST-GTPases (2 µM) were ADP-ribosylated by C3cer (5 nM final, 5 min at 37°C) in the presence of [³²P] NAD. Radiolabeled probes were subjected to SDS-PAGE and the autoradiography is shown.
Fig. 5 C) shows the alignment of partial sequence of RhoA, B, C and Rac1. Numbers indicate the position of the residues in the whole protein. Residues, which have been identified in RhoA to be critical for ADP-ribosylation, are boxed in Rac1. An arrow marks the acceptor amino acid Rho Asn41 in the C3-catalyzed ADP-ribosylation.
Fig. 5 D) shows the Kₘ for NAD in the C3cer catalyzed ADP-ribosylation of RhoA, B and C and specific ADP-ribosyltransferase activities of C3cer either for RhoA, B or C or RhoC 143V.

Fig. 6 A) shows the time dependent ADP-ribosylation of recombinant RhoA by C3cer wild type and mutant C3cer. 2 µM RhoA was incubated with the indicated toxins in the presence of [³²P]NAD for up to 4 min. Radio labeled probes were subjected to SDS-PAGE and the amount of modified RhoA was quantified by phosphor-imaging. The concentrations of wild- type (WT) C3cer were 10 nM and 1 µM for C3cer Y180T, Q183E and E185Q.
Fig. 6 B) shows the precipitation of C3cer WT and C3cer mutants with GST-RhoA immobilized to glutathione Sepharose beads. 1 µg of GST or 2 µg of GST-RhoA were incubated with either 300 ng of C3cer WT or mutant C3cer for 45 min at 4°C as described in Example 7. Bound toxin was detected with a specific polyclonal antibody against C3cer WT. Con: C3cer as a control for the antibody.

Fig. 7 A) shows the cytotoxic effects of C2IN-C3cer fusiontoxin on Vero cells. The cells were grown in DMEM plus 10% foetal calf serum (FCS) to sub-confluency. The medium was changed to DMEM without serum, proteins or (a) buffer as a control were added and cells were further incubated at 37°C. After 3 hours, cells were photographed. Proteins used are: (b) 500 ng/ml activated C2II (C2IIa) alone, (c) 200 ng/ml C2IIa plus 100 ng/ml C2IN-C3cer, (d) 200 ng/ml C2IIa plus 200 ng/ml C2IN-C3cer, (e) 500 ng/ml C2IIa plus 500 ng/ml C2IN-C3cer and (f) 500 ng/ml C2IN-C3cer alone.
Fig. 7 B) shows Vero cells, similar treated as described above, which were scraped from a 3 cm culture dish, washed and lysed by sonication. 50 µg of total cell lysate was [³²P]-ADP-ribosylated in the presence of 10 nM C2IN-C3cer and the probes were subjected to SDS-PAGE. The autoradiography is shown.

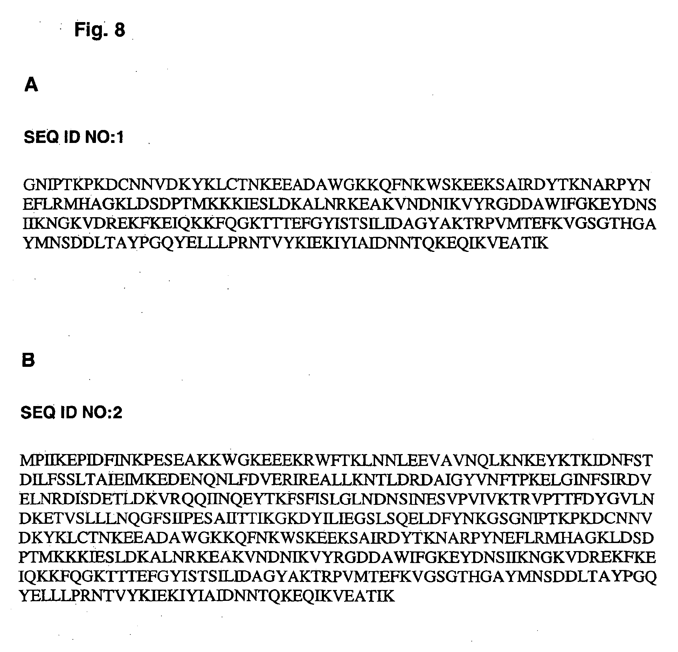

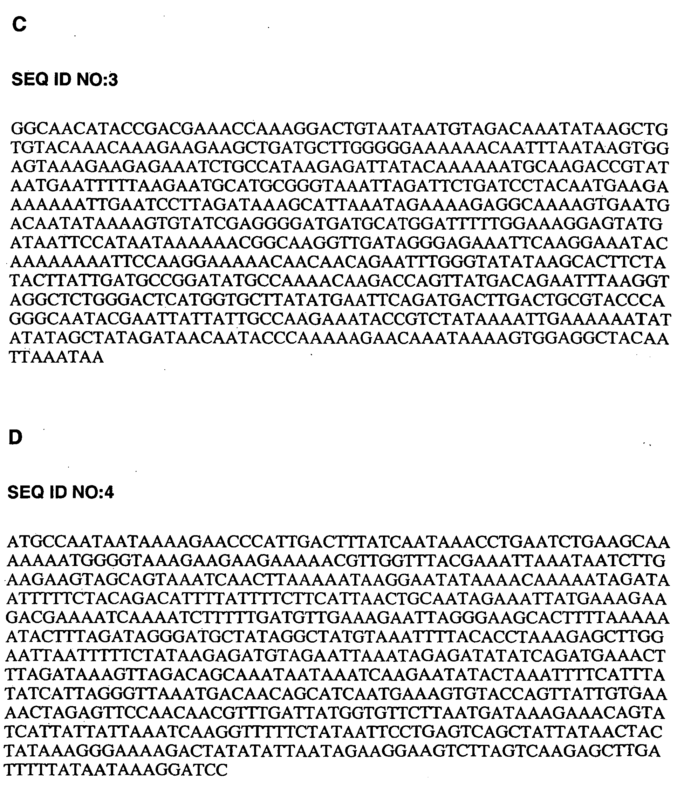

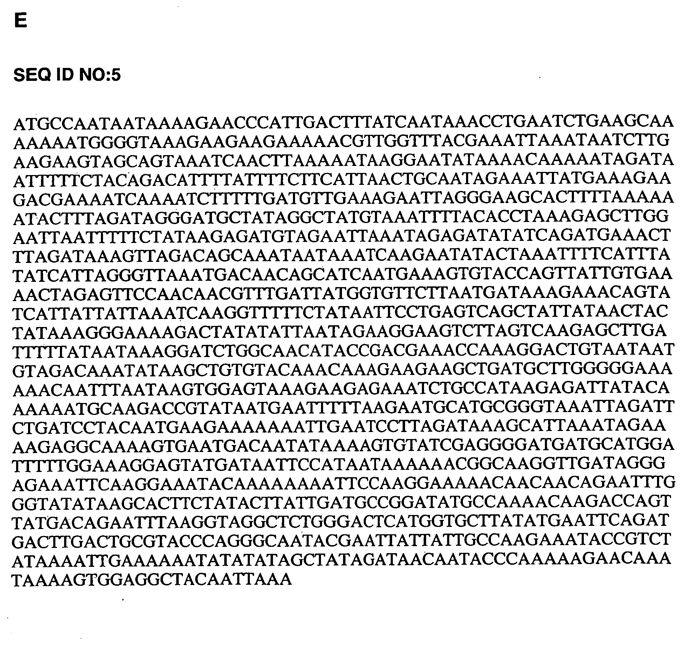

Fig. 8 A) shows the amino acid sequence SEQ ID NO: 1
Fig. 8 B) shows the amino acid sequence SEQ ID NO:2
Fig. 8 C) shows the nucleic acid sequence SEQ ID NO:3
Fig. 8 D) shows the nucleic acid sequence SEQ ID NO:4
Fig. 8 E) shows the nucleic acid sequence SEQ ID NO: 5
Fig. 9 demonstrates axonal outgrowth from retina miniexplants using C2C3cer and C2C3lim, respectively.

The following examples are thought to illustrate the invention, but not to limit the scope of the invention thereon.

### Examples

### Material and Chemicals

The oligonucleotides were obtained from MWG (Ebersberg, Germany). The pGEX-2T vector and the glutathione S-transferase Gene Fusion system were obtained from Pharmacia Biotech (Uppsala, Sweden), the Quick-Change Kit from Stratagene (Heidelberg, Germany). The restriction endonucleases were obtained from NEB (Schwalbach, Germany) and [³²P]-NAD was obtained from NEN (Belgium). All other chemicals were obtained from commercial sources.

### SDS-Polyacrylamide gel electrophoresis

All SDS-Polyacrylamide gel electrophoresis were performed according to the method of Laemmli (Laemmli, U K, 1970, Nature 227, 680-685). Gels were stained with Coomassie Brilliant Blue R250, dried and further analyzed.

### Example 1:

### Cloning of the recombinant C3cer ADP-ribosyltransferase

Before starting the experiment an alignment of the peptides derived from protease-digested, native ADP-ribosyltransferase from *B. cereus* (Just, I. et al., 1995, Biochemistry 34, 334-340) with other known C3-like ADP-ribosyltransferases was executed. The result showed that peptide 4 (amino acid sequence VGSGTHGAYMNSDDLTAYPGQYELLLPR) is probably located in the C-terminal part of the transferase.

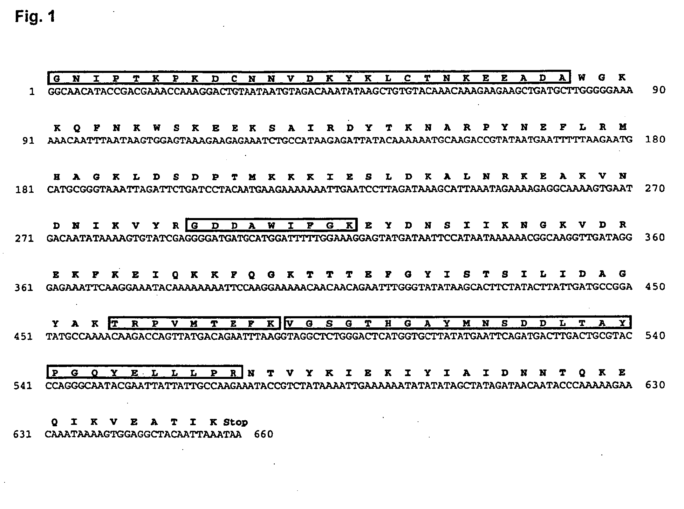

A first primer related to the N-terminus of the ADP-ribosyltransferase and a second primer related to the C-terminally located glutamate of peptide 4 were designed. Both primers were constructed in consideration of the codon-usage of several *Bacillus* strains. Using Taq-polymerase (NEB, Germany), a 567 bp fragment was amplified from genomic DNA of *Bacillus cereus* strain 2339. The fragment was subcloned into the pCR2.1 vector (Invitrogen, The Netherlands) and sequenced with the Cycle Sequencing Ready Reaction Kit (Perkin Elmer, USA). For determination of the remaining base pairs, genomic DNA from *B. cereus* 2339 was totally digested with Nsi I and separated on an agarose gel. The fragments were extracted with the JetSorb Kit (Genomed, Germany) and ligated in a Pst I digested pUC18 vector. PCR was performed with a specific pUC18 primer and a second primer, which was specific for the nucleotide sequence of the ADP-ribosyltransferase, covering base pairs 486-506 (5'-GGTAGGC TCTGGGACTCATGG-3'). PCR products were subcloned into pCR 2.1 TOPO-TA vector and sequenced. By this method, we identified one clone carrying the remaining 105 bp, followed by an in frame stop-codon. The complete coding sequence of the mature ADP-ribosyltransferase was amplified from genomic DNA and cloned into the pGEX 2TGL vector, followed by DNA-sequencing. The result is shown in Fig. 1.

### Example 2:

### Expression of the recombinant ADP-ribosyltransferases

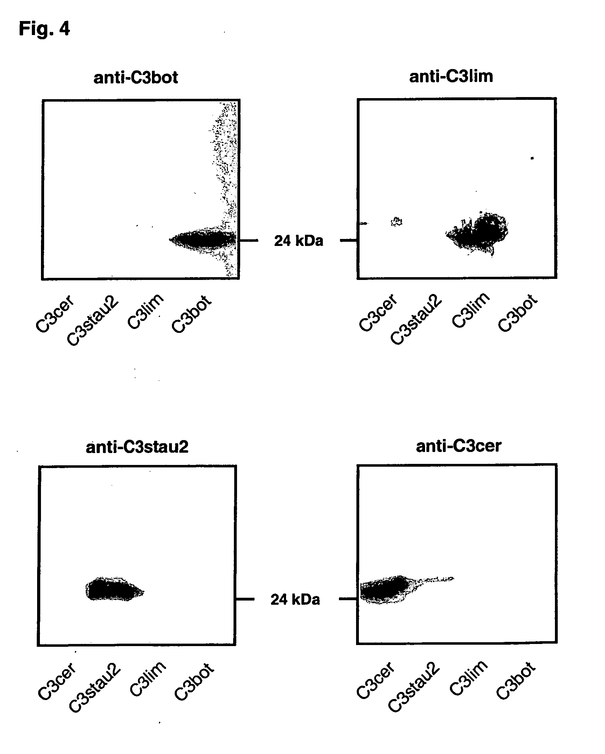

For expression of the recombinant C3cer wild type ADP-ribosyltransferase as glutathione S-transferase fusion protein, the pGEX 2TGL-C3cer vector was transformed into *E. coli* TG1 cells by standard methods. 11 LB medium containing 100 µg/ml ampicillin was inoculated with 30 ml of an overnight culture and cells were grown at 37°C until the optical density at 600 nm reaches 0.8. IPTG was added to a final concentration of 0.2 mM and cells were grown for another 6 h at 37°C. Cells were collected by centrifugation, resuspended in lysis buffer (50 mM HEPES [pH 7.5] and 1 mM PMSF) and broken with a French press (SLM Aminco, Spectronic Instruments). The lysate was centrifuged, glutathione-Sepharose beads were added to the supernatant and incubated for 45 min at 4°C. Beads were washed with 100fold beads volume wash buffer (50 mM HEPES [pH 7.5] and 150 mM NaCl). GST-C3 was eluted with a buffer containing 10 mM reduced glutathione, 50 mM HEPES [pH 7.5] and 100 mM NaCl. The GST-carrier was cleaved with thrombin followed by its removal with benzamidine beads. C3bot, C3lim, C3stau1 and 2 were purified as described elsewhere (Aktories, K et al., 1987, FEBS Lett. 212, 109-113; Just, I. et al., 1992, J. Biol. Chem. 267, 10274-10280; Wilde, C et al., 2001, J. Biol. Chem. 276, 9537-9542). All purification steps were checked by SDS-PAGE. The results are shown in Fig. 4.

### Example 3:

### Construction and expression of mutant C3cer

The following C3cer mutants were constructed:
**C19A** : amino acid cysteine at position 19 was replaced by amino acid alanine.
**R59K** : amino acid arginine at position 59 was replaced by amino acid lysine.
**R97K** : amino acid arginine at position 97 was replaced by amino acid lysine.
**Y151V :** amino acid threonine at position 151 was replaced by amino acid valine.
**R155E** : amino acid arginine at position 155 was replaced by amino acid glutamic acid.
**T178A** : amino acid threonine at position 178 was replaced by amino acid alanine.
**Y180T** : amino acid tyrosine at position 180 was replaced by amino acid threonine.
**Q183E** : amino acid glutamine at position 183 was replaced by amino acid glutamic acid.
**E185Q** : amino acid glutamic acid at position 185 was replaced by amino acid glutamine.
These C3cer mutants were constructed by site-directed mutagenesis with the pGEX-2T-C3cer plasmid as a template and the respective oligonucleotides using the Quick-Change Kit according to the manufacturer's instructions. From two complementary primers needed, only one is indicated: **C19A,** 5'-CAA ATA TAA GCT GGC TAC AAA CAA AG-3'; **R59K,** 5'-GAA TTT TTA AAA ATG CAT GCG G-3'; **R97K** 5'-TAT AAA AGT GTA TAA AGG GGA TGA TGC-3'; **T151V,** 5'-ATT GAT GCC GGA GTT GCC AAA ACA AGA CC-3', **R155E,** 5'-TAT GCC AAA ACA GAA CCA GTT ATG ACA GAA-3'; **T178A,** 5'-CAG ATG ACT TGG CTG CGT ACC CAG-3'; **Y180T,** 5'-GAC TTG ACT GCG ACA CCA GGG CAA TAC G-3'; **Q183E,** 5'-CGT ACC CAG GGG AAT ACG AAT TAT TAT TG-3'; **E185Q,** 5'-CCA GGG CAA TAC CAA TTA TTA TTG C-3'. Mutated plasmids were transformed in *E. coli* TG1 cells and all mutations were confirmed by DNA sequencing. Proteins were expressed as thrombin-cleaved proteins as described above.

### Example 4:

### ADP-ribosylation reaction of several C3-like ADP-ribosyltransferases

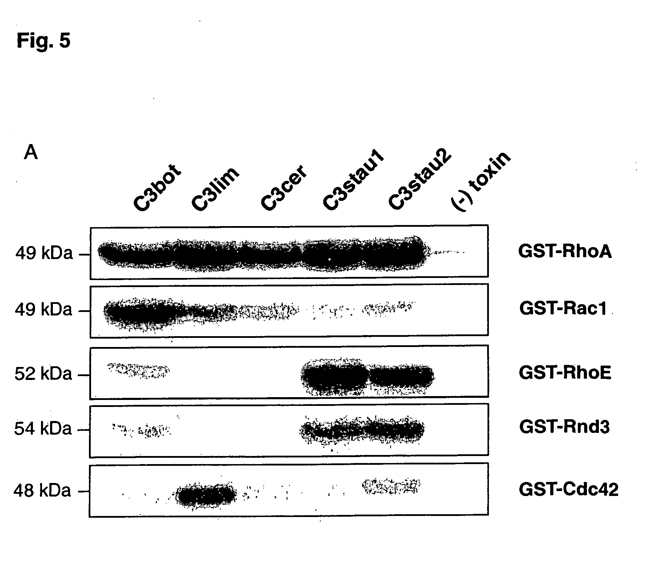

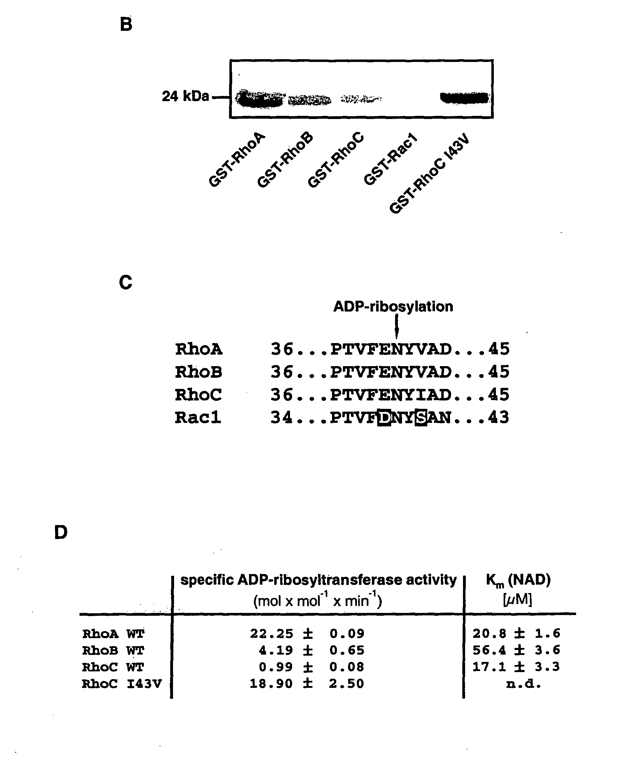

2 µM recombinant RhoA, Racl, RhoE, Rnd3 or Cdc42 were incubated with C3bot, C3lim, C3stau1, C3stau2 or C3cer, respectively at various concentrations in a buffer containing 50 mM HEPES (pH 7.3), 2 mM MgCl₂ and various concentrations of [adenylate-³²P] NAD at 37°C as indicated. Radiolabeled proteins were analyzed by phosphor-imaging and the amount of incorporated [³²P] ADP-ribose was calculated from phosphor-imager data. The results are shown in Fig. 5 A.

### Example 5:

### ADP-ribosylation reaction of C3cer ADP-ribosyltransferase

2 µM recombinant RhoA, RhoB, RhoC or RhoC I43V were incubated with C3cer wild type or mutant protein at various concentrations in a buffer containing 50 mM HEPES (pH 7.3), 2 mM MgCl₂ and various concentrations of [adenylate-³²P] NAD at 37°C as indicated. Radiolabeled proteins were analyzed by phosphor-imaging and the amount of incorporated [³²P] ADP-ribose was calculated from phosphor-imager data. The results are shown in Fig. 5 B

### Example 6:

### NAD glycohydrolase reaction

For detection of glycohydrolase activity, 200 µM [adenylate- ³²P] NAD were incubated with various concentrations of either C3cer wild type or mutant C3cer in a buffer containing 50 mM HEPES (pH 7.3) and 2 mM MgCl₂ at 37°C for up to 3h. Three µl aliquots of the reaction mix were separated by TLC on Silica Gel 60_{F254} (Merck, Germany) with 66% 2-propanol and 0.33% ammonium sulfate, followed by phosphor-imaging. The amount of cleaved [adenylate-³²P] ADP-ribose was calculated from phosphor-imager data. The results are shown in Table 1 below.

### Example 7:

### Precipitation assay

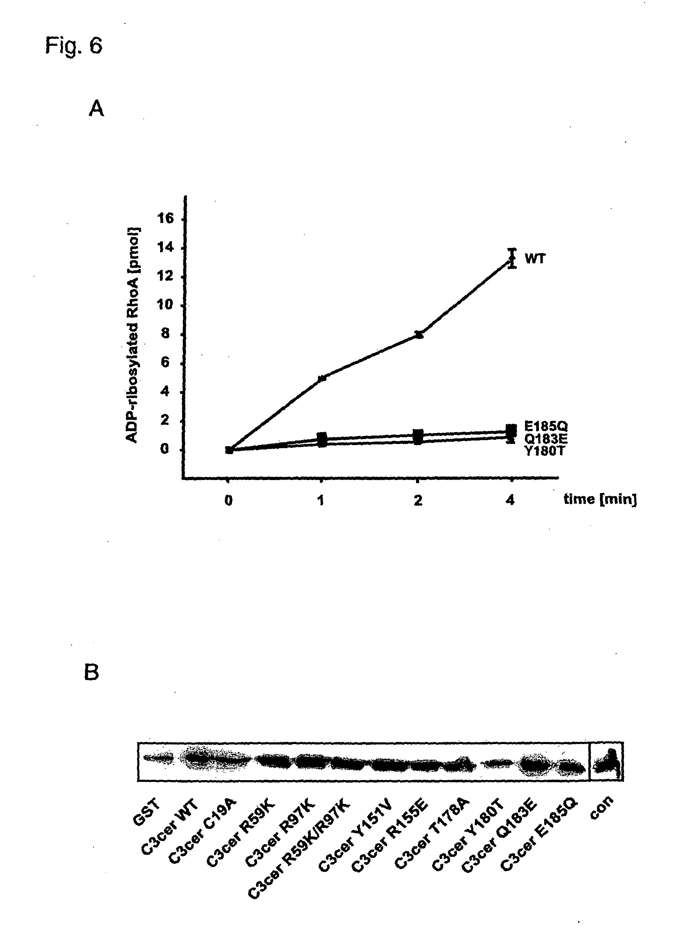

Wild type RhoA was expressed as recombinant glutathione S-transferase fusion protein in *E. coli* and coupled to glutathione beads. 2 µg of RhoA was incubated with 300 ng of toxin (C3cer wild type or mutant as indicated) in a buffer containing 50 mM HEPES (pH 7.3), 2 mM MgCl₂, 1 mM DTT, 1 mM EDTA, 1 mM PMSF and Nonidet P40 (final concentration 0.5%) for 45 min at 4°C. Beads were washed 3 times with buffer and subjected to SDS-PAGE. After transferring proteins onto nitrocellulose, bound toxin was detected with a specific polyclonal antibody against the C3cer wild type protein. 200ng C3cer was used as control. The results are shown in Fig. 6 B.

### Example 8:

### Western-Blotting analysis

Recombinant C3-like ADP-ribosyltransferases were expressed and purified as described elsewhere (Aktories, K et al., 1987, FEBS Lett. 212, 109-113; Just, I. et al., 1992, J. Biol. Chem. 267, 10274-10280; Wilde, C et al., 2001, J. Biol. Chem. 276, 9537-9542). Antisera against C3bot, C3lim, C3stau2 and C3cer (each 1:10000 diluted in PBST) were obtained from rabbit. The second antibody was a goat-anti-rabbit antibody coupled to horseradish peroxidase and visualization was performed using the ECL system. The results are shown in Fig. 4.

### Example 9:

### Construction of C2IN-C3cer fusion toxin

The C2IN-C3cer fusion toxin was constructed as described (Barth, H et al., 1998, Infect. Immun. 66, 1364-1369) using the Bgl II / BamH HI site flanking the ADP-ribosyltransferase gene. The recombinant fusion toxin was expressed as glutathione S-transferase fusion protein and purified as thrombin-cleaved recombinant protein. Purification was checked by SDS-PAGE.

### Example 10:

### Cytotoxicity assay

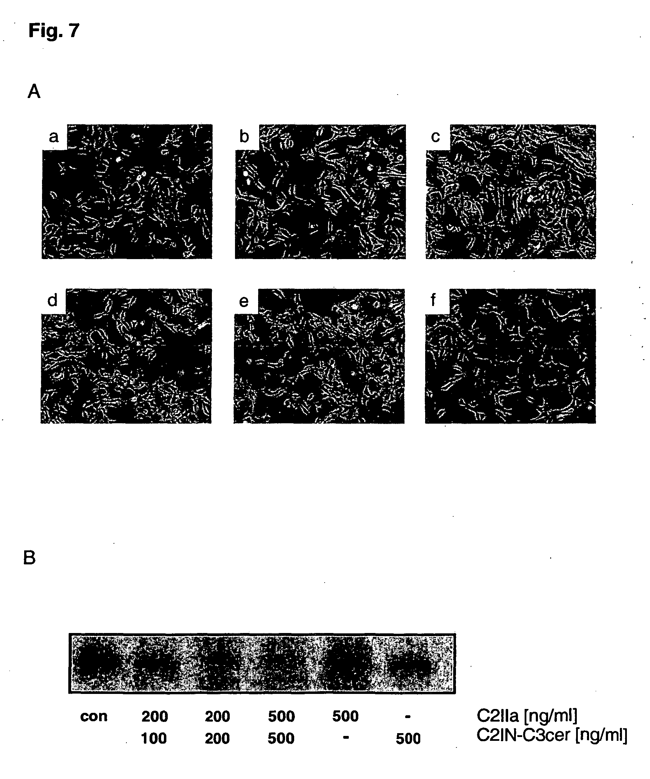

Vero cells were grown to sub-confluency at 37°C, 5% CO₂ in Dulbecco's minimum essential medium (DMEM) containing 10% FCS, 2 mM L- glutamate, 100 U of penicillin/ ml and 100 µg/ml streptomycin. For cytotoxic assays, medium was changed to serum-free DMEM and sub-confluent cells were treated with either activated C2II toxin or C2IN-C3cer alone or with activated C2II in combination with increasing concentrations of C2IN-C3cer. After 3 h, the cells were photographed. The results are shown in Fig. 7A.

For differential ADP-ribosylation, vero cells were treated as described above. The cells were washed three times with PBS, scraped off the plates and lysed by sonication. 50 µg lysate (Bradford) were used for ADP-ribosylation by C2IN-C3cer as described above. The results are shown in Fig. 7B.

The specific ADP-ribosyltransferase- and NAD-glycohydrolase activities were measured as described above and determined as 22.25 mol x mol⁻¹ toxin x min⁻¹ for transferase- and 0.29 mol hydrolyzed [³²P] NAD x mol⁻¹ toxin x min⁻¹ for glycohydrolase activity, respectively. For calculation of the relative enzyme activities, wild type activity was set as 100% and S.E. is given from three independent experiments. For immunoprecipitation experiments, glutathione Sepharose beads-coupled RhoA was incubated with the indicated proteins. Beads were washed, subjected to SDS-PAGE and blotted onto nitrocellulose. Bound toxins were detected with a specific polyclonal C3cer antibody. +: normal binding to RhoA, (+): reduced binding to RhoA. Data are summarized from Fig. 6B.

### Example 11:

### Neurite outgrowth on inhibitory CSPG

### Materials

- Chicken eggs, embryonic development E7 (Weiß, Kirchberg, Germany)
- Hanks Balanced Salt Solution (HBSS) with Ca & Mg with Phenol Red (PAA Laboratories, Cölbe, Germany)
- PBS, pH 7.4 without Ca & Mg (PAA Laboratories, Cölbe, Germany)
- Culture medium: Ham's F-12 without L-glutamine (PAA Laboratories, Cölbe, Germany), supplemented with 10% FCS (fetal calf serum), 2% chicken serum, 2 mM L-glutamine, 100 units/ml Penicillin, 100 µg/ml Streptomycin (all components provided from PAA Laboratories, Cölbe, Germany
- Poly-L-Lysine (Sigma, Taufkirchen, Germany)
- Chondroitinsulfate Proteoglycan (CSPG; Chemicon, Hofheim, Germany)
- Laminin (Becton Dickinson, Heidelberg, Germany)
- Glass cover slips, round, 13 mm diameter (Assistent)
- Cell culture plastic ware: 24-well cell culture plates (Nunc, Wiesbaden, Germany)
- Triton X-100 (Roth, Karlsruhe, Germany)
- Alexa Flour 488 phalloidin (Molecular Probes, Leiden, Netherlands)
- Alexa Phalloidin staining solution: Alexa Flour 488 phalloidin 1:40 in PBS containing 1% BSA and 0.02% NaAzide
- BSA (Bovine Serum Albumin Fraktion V; Roth)
- Sodium azide (Merck, Bruchsal, Germany)
- Glycerin (Roth, Karlsruhe, Germany)
- Tissue chopper (McIlwain Tissue Chopper, Intracel)
- Mowiol embedding solution (Hoechst, Frankfurt, Germany)
- MorphoChem test substances: compounds were delivered in 20mM stock solutions (solved in 100% DMSO,) in 96-well plates on dry ice from MorphoChem AG, Munich, Germany. These stock solutions were stored at - 80 ± 15°C.

### Methods

### Coating of cover slips:

Prior to coating cover slips were sterilized under UV light, every side for 10-20 min. Cover slips were precoated with Poly-L-Lysine (200 µg/ml, dissolved in PBS, pH 7.4) at 37°C for 2-3 hours, then washed once with PBS and stored overnight in PBS at 2-8 °C. Cover slips were then coated with a CSPG-Laminin mixture (20 µg/ml CSPG diluted in a 20 µg/ml Laminin solution) at 37°C for 2-3 hours.

Just prior to the explantation of retinal explants, cover slips were dispersed into wells of a 24-well tissue culture plate, washed once with HBSS and used immediately.

### Preparation of retinal mini explants:

Chicken eggs of embryonic development E7 were cleaned with 70% EtOH. After taking out the embryo, eyes were resected in HBSS and the retina prepared. The isolated retina was placed onto the polyamide plate of a tissue chopper and was cut into 300 µm x 300 µm squares.

The retinal mini explants were transfered to a petri dish with culture medium. 20-30 mini explants were seeded into each well of a 24-well plate containing the coated cover slips in a total volume of 500 µl per well. Before adding the reaction mixtures, retinal mini explants were incubated at 37°C, 4% CO₂ for 30 min to allow the mini explants sufficient time for adherence to the cover slips.

### Incubation with C2C3cer and C2C3lim:

C2C3cer and C2C3lim were diluted in 500 µl prewarmed (37°C) culture medium in sterile reaction tubes, vortexed for 2-3 sec, and then added to the mini explants (final volume: 1ml per well). C2C3cer and C2C3lim were used in following concentrations: 0,1, 1, and 10 µg/ml. Negative controls received culture medium only. After ensuring that retinal mini explants were distributed evenly, cultures were incubated for 24-28 hours at 37°C, 4% CO₂.

### Staining with Alexa-Phalloidin:

Retinal cultures were fixed overnight at 2-8°C by adding 1 ml of 4% PFA (in PBS, pH 7.4) to each well. After washing with 1 ml PBS per well (2 x for 5 min), cultures were incubated with Triton X-100 (0.1% in PBS) for 10 min at room temperature and then washed again (3 x for 5 min). To stain the outgrowing neurites of retinal mini explants, 300 µl Alexa Phalloidin staining solution was added to each well for 30 min at room temperature. Cultures were washed once again (3 x for 5 min) and embedded in Mowiol.

### Analysis of neurite outgrowth:

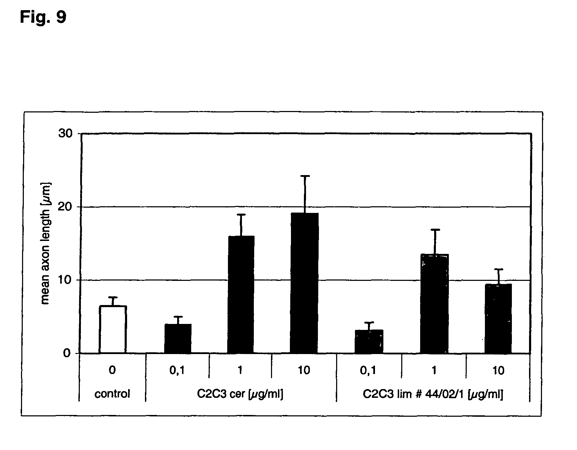

Neurite outgrowth of each explant was analyzed with help of a digital camera (Axiocam, Zeiss) attached to a fluorescence microscope (Axioplan 2, Zeiss). With use of a measuring software (Axio Vision 3.0, Zeiss), axon length of each explant was determined at four different defined points around the explant and the mean axon length per explant was calculated. The results are given in Table 2 and Fig. 9.

This example demonstrates that C2C3cer in concentrations of 1 and 10 µg/ml provokes an outgrowth of axons from retina miniexplants. The outgrowth is more pronounced in experiments using C2C3cer than in experiments using C2C3lim.

**Table 1:**

| **Summary of properties of C3cer wild type (WT) and C3cer mutants** | | | |
|---|---|---|---|
| Toxin | ADP-ribosyltransferase activity [relative activity in %] | NAD glycohydrolase activity [relative activity in %] | RhoA binding |
| C3cer WT | 100 | 100 | + |
| C3cer C19A | 118 ±0.09 | 197 ±10.8 | |
| C3cer R59K | 1.1 ± 0.09 | <0.01 | + |
| C3cer R97K | 1.9 ± 0.05 | <0.01 | + |
| C3cer R59K/R97K | <0.01 | <0.01 | + |
| C3cer T151V | 1.6 ± 0.08 | 4.1 ±0.03 | + |
| C3cer R155E | 0.2 ± 0.001 | <0.01 | + |
| C3cer T178A | 1.3 ±0.2 | 52 ±1.4 | + |
| C3cer T178A/Q183E | <0.01 | 29 ±2.0 | n.d. |
| C3cer Y180T | <0.01 | 545 ±10.4 | (+) |
| C3cer Q183E | <0.01 | 45 ±3.5 | + |
| C3cer E185Q | <0.01 | <0.01 | + |

**Table 2:**

| **Neurite outgrowth of retina miniexplants using C2C3cer and C2C3lim.** | | | | |
|---|---|---|---|---|
| | **Concentration [*µ*g/ml]** | **Mean axon Mean axon length [*µ*m]** | **S. E. M. [*µ*m]** | **n** |
| **Control** | 0 | 6,4 | 1,2 | 71 |
| **C2C3 cer** | 0,1 | 3,9 | 1,1 | 48 |
| | 1 | 15,9 | 3,0 | 48 |
| | 10 | 19,1 | 5,1 | 28 |
| **C2C3 lim # 44/02/1** | 0,1 | 3,1 | 1,1 | 42 |
| | 1 | 13,5 | 3,4 | 12 |
| | 10 | 9,4 | 2,1 | 47 |

## Claims

1. An ADP-ribosyltransferase C3cer.

2. The ADP-ribosyltransferase C3cer of claim 1, wherein the ADP-ribosyltransferase C3cer selectively ADP-ribosylates RhoA, RhoB and/or RhoC.

3. The ADP-ribosyltransferase C3cer of any of claim 1 or 2, wherein the ADP-ribosylation inhibits RhoA, RhoB and/or RhoC.

4. The ADP-ribosyltransferase C3cer of any of claims 1 to 3 containing or consisting of the amino acid sequence of SEQ ID NO: 1.

5. A fusion protein containing the ADP-ribosyltransferase C3cer of any of claims 1 to 4 and at least a second protein.

6. The fusion protein of claim 5, wherein the second protein mediates the incorporation of the fusion protein into a target cell.

7. The fusion protein of claim 6, wherein the second protein binds directly to the target cell.

8. The fusion protein of claim 6, wherein the second protein binds via a transporter to the target cell.

9. The fusion protein of claim 7 or 8, wherein the second protein or the transporter binds to a structure on the surface of the target cell, particularly to a receptor or a surface protein.

10. The fusion protein of claims 7 to 9, wherein the second protein contains the enzyme component of a binary bacterial toxin or parts thereof, preferred the C2I component, more preferred the C2IN component of the C2 toxin, preferred of the C2 toxin from *Clostridium botulinum.*

11. The fusion protein of any of claims 8 to 10, wherein the transporter contains the binding component of a binary bacterial toxin or parts thereof, preferred the C2II component of the C2 toxin, preferred of the C2 toxin from *Clostridium botulinum.*

12. The fusion protein of claim 6, wherein the second protein is the diphtheria toxin from *Corynebacterium diphteriae,* the pseudomonas exotoxin A from *Pseudomonas aeruginosum.*

13. The fusion protein of any of claims 5 to 12, wherein the fusion protein selectively ADP-ribosylates RhoA, RhoB and/or RhoC.

14. The fusion protein of any of claims 5 to 13, wherein the ADP-ribosylation inhibits RhoA, RhoB and/or RhoC.

15. The fusion protein of any of claims 5 to 14 containing or consisting of the amino acid sequence of SEQ ID NO: 2.

16. A nucleic acid encoding the ADP-ribosyltransferase C3cer of any of claims 1 to 4 or the fusion protein of any of claims 5 to 15.

17. The nucleic acid of claim 16 containing or consisting of the nucleic acid sequence SEQ ID NO:3.

18. The nucleic acid of claim 16 containing or consisting of the nucleic acid sequence SEQ ID NO:4.

19. The nucleic acid of claim 16 containing or consisting of the nucleic acid sequence SEQ ID NO:5.

20. Use of the ADP-ribosyltransferase C3cer of any of claims 1 to 4 or of the fusion protein of any of claims 5 to 15 for the preparation of a medicament for the regeneration of neurons and/or for the stimulation of the growth of neurons.

21. Use of claim 20, when the regeneration of neurons and/or the stimulation of growth of neurons is in the context of neurological and/or neurodegenerative diseases of the peripheral and/or central nervous system.

22. Use of claim 21, wherein the disease is selected of the group comprising:
stroke, multiple sclerosis, HIV dementia, Parkinson's disease, Alzheimer's disease, ischemia, epilepsy, Morbus Huntington AIDS, neuronal regeneration after tumor extirpation, prion disease or other diseases of the CNS where axons are damaged in the CNS environment, diabetic neuropathy, cancer, metastasis, hypertension, cardiac diseases, hyperalgesia, neuropathy, eye diseases such as glaucoma, penile erectile dysfunction. Furhtermore the treatment with Rho antagonists would be used to enhance the rate of axon growth of peripheral nerves and thereby be effective for repair of peripheral nerves after surgery.

23. Use of claim 20, when the regeneration of neurons and/or the stimulation of growth of neurons is in the context of lesions of the cerebric and/or spinal cord.

24. A method for the production of a production cell comprising the insertion of at least one nucleic acid of any of claims 16, 17 or 19 into a cell.

25. A production cell obtainable by the method of claim 24.

26. A method for the production of the ADP-ribosyltransferase C3cer of any of claims 1 to 4 or of the fusion protein of any of claims 5 to 15, comprising the following steps:
a. cultivating a production cell of claim 25 and expressing the nucleic acid under suitable conditions, and
b. isolating the ADP-ribosyltransferase C3cer or the fusion protein with suitable means.

27. A pharmaceutical composition containing the ADP-ribosyltransferase C3cer of any of claims 1 to 4 and/or a fusion protein of any of claims 5 to 15 and suitable adjuvants and/or pharmaceutically acceptable carriers.

28. A method for the production of a pharmaceutical composition of claim 27 comprising the following steps:
a. performing the method of claim 26, and
b. combining the ADP-ribosyltransferase C3cer and/or the fusion protein with suitable adjuvants and/or pharmaceutically acceptable carriers.


SEQUENCE LISTING

<110> Migragen AG

<120> ADP-ribosyltransferase C3cer

<130> M37224EP

<160> 5

<170> PatentIn version 3.1

<210> 1
<211> 219
<212> PRT
<213> Bacillus cereus

<400> 1

Gly Asn Ile Pro Thr Lys Pro Lys Asp Cys Asn Asn Val Asp Lys Tyr
1               5                   10                  15

Lys Leu Cys Thr Asn Lys Glu Glu Ala Asp Ala Trp Gly Lys Lys Gln
            20                  25                  30

Phe Asn Lys Trp Ser Lys Glu Glu Lys Ser Ala Ile Arg Asp Tyr Thr
        35                  40                  45

Lys Asn Ala Arg Pro Tyr Asn Glu Phe Leu Arg Met His Ala Gly Lys
    50                  55                  60

Leu Asp Ser Asp Pro Thr Met Lys Lys Lys Ile Glu Ser Leu Asp Lys
65                  70                  75                  80

Ala Leu Asn Arg Lys Glu Ala Lys Val Asn Asp Asn Ile Lys Val Tyr

85                  90                  95

Arg Gly Asp Asp Ala Trp Ile Phe Gly Lys Glu Tyr Asp Asn Ser Ile
            100                 105                 110

Ile Lys Asn Gly Lys Val Asp Arg Glu Lys Phe Lys Glu Ile Gln Lys
        115                 120                 125

Lys Phe Gln Gly Lys Thr Thr Thr Glu Phe Gly Tyr Ile Ser Thr Ser
    130                 135                 140

Ile Leu Ile Asp Ala Gly Tyr Ala Lys Thr Arg Pro Val Met Thr Glu
145                 150                 155                 160

Phe Lys Val Gly Ser Gly Thr His Gly Ala Tyr Met Asn Ser Asp Asp
                165                 170                 175

Leu Thr Ala Tyr Pro Gly Gln Tyr Glu Leu Leu Leu Pro Arg Asn Thr
            180                 185                 190

Val Tyr Lys Ile Glu Lys Ile Tyr Ile Ala Ile Asp Asn Asn Thr Gln
        195                 200                 205

Lys Glu Gln Ile Lys Val Glu Ala Thr Ile Lys
    210                 215

<210>  2

<211>  445

<212>  PRT

<213>  Bacillus cereus

<400>  2

Met Pro Ile Ile Lys Glu Pro Ile Asp Phe Ile Asn Lys Pro Glu Ser
1               5                   10                  15

Glu Ala Lys Lys Trp Gly Lys Glu Glu Glu Lys Arg Trp Phe Thr Lys
            20                  25                  30

Leu Asn Asn Leu Glu Glu Val Ala Val Asn Gln Leu Lys Asn Lys Glu
        35                  40                  45

Tyr Lys Thr Lys Ile Asp Asn Phe Ser Thr Asp Ile Leu Phe Ser Ser
    50                  55                  60

Leu Thr Ala Ile Glu Ile Met Lys Glu Asp Glu Asn Gln Asn Leu Phe
65                  70                  75                  80

Asp Val Glu Arg Ile Arg Glu Ala Leu Leu Lys Asn Thr Leu Asp Arg
                85                  90                  95

Asp Ala Ile Gly Tyr Val Asn Phe Thr Pro Lys Glu Leu Gly Ile Asn
            100                 105                 110

Phe Ser Ile Arg Asp Val Glu Leu Asn Arg Asp Ile Ser Asp Glu Thr
        115                 120                 125

Leu Asp Lys Val Arg Gln Gln Ile Ile Asn Gln Glu Tyr Thr Lys Phe
    130                 135                 140

Ser Phe Ile Ser Leu Gly Leu Asn Asp Asn Ser Ile Asn Glu Ser Val
145                 150                 155                 160

Pro Val Ile Val Lys Thr Arg Val Pro Thr Thr Phe Asp Tyr Gly Val
                165                 170                 175

Leu Asn Asp Lys Glu Thr Val Ser Leu Leu Leu Asn Gln Gly Phe Ser
            180                 185                 190

Ile Ile Pro Glu Ser Ala Ile Ile Thr Thr Ile Lys Gly Lys Asp Tyr
        195                 200                 205

Ile Leu Ile Glu Gly Ser Leu Ser Gln Glu Leu Asp Phe Tyr Asn Lys
    210                 215                 220

Gly Ser Gly Asn Ile Pro Thr Lys Pro Lys Asp Cys Asn Asn Val Asp
225                 230                 235                 240

Lys Tyr Lys Leu Cys Thr Asn Lys Glu Glu Ala Asp Ala Trp Gly Lys
                245                 250                 255

Lys Gln Phe Asn Lys Trp Ser Lys Glu Glu Lys Ser Ala Ile Arg Asp
            260                 265                 270

Tyr Thr Lys Asn Ala Arg Pro Tyr Asn Glu Phe Leu Arg Met His Ala
        275                 280                 285

Gly Lys Leu Asp Ser Asp Pro Thr Met Lys Lys Lys Ile Glu Ser Leu
    290                 295                 300

Asp Lys Ala Leu Asn Arg Lys Glu Ala Lys Val Asn Asp Asn Ile Lys
305                 310                 315                 320

Val Tyr Arg Gly Asp Asp Ala Trp Ile Phe Gly Lys Glu Tyr Asp Asn
                325                 330                 335

Ser Ile Ile Lys Asn Gly Lys Val Asp Arg Glu Lys Phe Lys Glu Ile
            340                 345                 350

Gln Lys Lys Phe Gln Gly Lys Thr Thr Thr Glu Phe Gly Tyr Ile Ser
        355                 360                 365

Thr Ser Ile Leu Ile Asp Ala Gly Tyr Ala Lys Thr Arg Pro Val Met
    370                 375                 380

Thr Glu Phe Lys Val Gly Ser Gly Thr His Gly Ala Tyr Met Asn Ser
385                 390                 395                 400

Asp Asp Leu Thr Ala Tyr Pro Gly Gln Tyr Glu Leu Leu Leu Pro Arg
                405                 410                 415

Asn Thr Val Tyr Lys Ile Glu Lys Ile Tyr Ile Ala Ile Asp Asn Asn
            420                 425                 430

Thr Gln Lys Glu Gln Ile Lys Val Glu Ala Thr Ile Lys
        435                 440                 445

<210>  3

<211>  660

<212>  DNA

<213>  Bacillus cereus

<400>  3

ggcaacatac cgacgaaacc aaaggactgt aataatgtag acaaatataa gctgtgtaca
60

aacaaagaag aagctgatgc ttgggggaaa aaacaattta ataagtggag taaagaagag
120

aaatctgcca taagagatta tacaaaaaat gcaagaccgt ataatgaatt tttaagaatg
180

catgcgggta aattagattc tgatcctaca atgaagaaaa aaattgaatc cttagataaa
240

gcattaaata gaaaagaggc aaaagtgaat gacaatataa aagtgtatcg aggggatgat
300

gcatggattt ttggaaagga gtatgataat tccataataa aaaacggcaa ggttgatagg
360

gagaaattca aggaaataca aaaaaaattc caaggaaaaa caacaacaga atttgggtat
420

ataagcactt ctatacttat tgatgccgga tatgccaaaa caagaccagt tatgacagaa
480

tttaaggtag gctctgggac tcatggtgct tatatgaatt cagatgactt gactgcgtac
540

ccagggcaat acgaattatt attgccaaga aataccgtct ataaaattga aaaaatatat
600

atagctatag ataacaatac ccaaaaagaa caaataaaag tggaggctac aattaaataa
660

<210> 4

<211> 678

<212> DNA

<213> bacillus cereus

<400> 4
atgccaataa taaaagaacc cattgacttt atcaataaac ctgaatctga agcaaaaaaa
60

tggggtaaag aagaagaaaa acgttggttt acgaaattaa ataatcttga agaagtagca
120

gtaaatcaac ttaaaaataa ggaatataaa acaaaaatag ataatttttc tacagacatt
180

ttattttctt cattaactgc aatagaaatt atgaaagaag acgaaaatca aaatcttttt
240

gatgttgaaa gaattaggga agcactttta aaaaatactt tagataggga tgctataggc
300

tatgtaaatt ttacacctaa agagcttgga attaattttt ctataagaga tgtagaatta
360

aatagagata tatcagatga aactttagat aaagttagac agcaaataat aaatcaagaa
420

tatactaaat tttcatttat atcattaggg ttaaatgaca acagcatcaa tgaaagtgta
480

ccagttattg tgaaaactag agttccaaca acgtttgatt atggtgttct taatgataaa
540

gaaacagtat cattattatt aaatcaaggt ttttctataa ttcctgagtc agctattata
600

actactataa agggaaaaga ctatatatta atagaaggaa gtcttagtca agagcttgat
660

ttttataata aaggatcc
678

<210> 5

<211> 1335

<212> DNA

<213> Bacillus cereus

<400> 5
atgccaataa taaaagaacc cattgacttt atcaataaac ctgaatctga agcaaaaaaa
60

tggggtaaag aagaagaaaa acgttggttt acgaaattaa ataatcttga agaagtagca
120

gtaaatcaac ttaaaaataa ggaatataaa acaaaaatag ataatttttc tacagacatt
180

ttatttcctt cattaactgc aatagaaatt atgaaagaag acgaaaatca aaatcttttt
240

gatgttgaaa gaattaggga agcactttta aaaaatactt tagataggga tgctataggc
300

tatgtaaatt ttacacctaa agagcttgga attaattttt ctataagaga tgtagaatta
360

aatagagata tatcagatga aactttagat aaagttagac agcaaataat aaatcaagaa
420

tatactaaat tttcatttat atcattaggg ttaaatgaca acagcatcaa tgaaagtgta
480

ccagttattg tgaaaactag agttccaaca acgtttgatt atggtgttct taatgataaa
540

gaaacagtat cattattatt aaatcaaggt ttttctataa ttcctgagtc agctattata
600

actactataa agggaaaaga ctatatatta atagaaggaa gtcttagtca agagcttgat
660

ttttataata aaggatctgg caacataccg acgaaaccaa aggactgtaa taatgtagac
720

aaatataagc tgtgtacaaa caaagaagaa gctgatgctt gggggaaaaa acaatttaat
780

aagtggagta aagaagagaa atctgccata agagattata caaaaaatgc aagaccgtat
840

aatgaatttt taagaatgca tgcgggtaaa ttagattctg atcctacaat gaagaaaaaa
900

attgaatcct tagataaagc attaaataga aaagaggcaa aagtgaatga caatataaaa
960

gtgtatcgag gggatgatgc atggattttt ggaaaggagt atgataattc cataataaaa
1020

aacggcaagg ttgataggga gaaattcaag gaaatacaaa aaaaattcca aggaaaaaca
1080

acaacagaat ttgggtatat aagcacttct atacttattg atgccggata tgccaaaaca
1140

agaccagtta tgacagaatt taaggtaggc tctgggactc atggtgctta tatgaattca
1200

gatgacttga ctgcgtaccc agggcaatac gaattattat tgccaagaaa taccgtctat
1260

aaaattgaaa aaatatatat agctatagat aacaataccc aaaaagaaca aataaaagtg
1320

gaggctacaa ttaaa
1335